# EUROPÄISCHE PATENTSCHRIFT

(11) **EP 2 550 347 B1**
(45) Veröffentlichungstag und Bekanntmachung des Hinweises auf die Patenterteilung: **14.06.2017**
(21) Anmeldenummer: 11710424.0
(22) Anmeldetag: 09.03.2011
(51) Int. Cl.: C11D 1/825, C11D 17/00, A61K 8/06

(54) **VERWENDUNG VON REINIGUNGSMITTELN ENTHALTEND WACHSHALTIGE MIKROEMULSIONEN**
USE OF CLEANING AGENTS CONTAINING MICROEMULSIONS THAT CONTAIN WAX
UTILISATION DE DÉTERGENTS CONTENANT DES MICRO-ÉMULSIONS À TENEUR EN CIRE

(30) Priorität: 23.03.2010 EP 10003051
(43) Veröffentlichungstag der Anmeldung: 30.01.2013
(73) Patentinhaber: Cognis IP Management GmbH, 40589 Dusseldorf (DE)
(72) Erfinder: HLOUCHA, Matthias, 50677 Köln (DE); KÜSTERS, Esther, 40625 Düsseldorf (DE); MENZER, Jasmin, 40789 Monheim (DE); PRINZ, Daniela, 41542 Dormagen (DE); HOLZ, Martina, 40764 Langenfeld (DE); ALBERS, Thomas, 40593 Düsseldorf (DE); SEIPEL, Werner, 40723 Hilden (DE); ERASMY, Jessica, 50823 Köln (DE); HENSEN, Hermann, 42781 Haan (DE); PELLÓN, Guadalupe, 40597 Düsseldorf (DE); CORNELSEN, Sybille, 40883 Ratingen (DE); BOYXEN, Norbert, 47906 Kempen (DE)
(74) Vertreter: BASF IP Association
(86) Internationale Anmeldenummer: PCT/EP2011/001136
(87) Internationale Veröffentlichungsnummer: WO 2011/116881

(56) Entgegenhaltungen:
- EP-A1- 2 368 972
- EP-A2- 0 559 304
- EP-A2- 0 813 861
- EP-A2- 1 152 051
- WO-A2-2008/155073

## Beschreibung

Die Erfindung befindet sich auf dem Gebiet der kosmetischen Reinigungsmittel, die in Form feinteiliger Emulsionen auf Basis ölhaltiger Mikroemulsionen vorliegen, welche Wachse enthalten. Reinigungsmittel stellen in der Regel mehr oder weniger konzentrierte Emulsionen dar, welche bekannter weise neben Ölkomponenten insbesondere Tenside und Pflegestoffe enthalten. Seitens des Verbrauchers besteht dabei der Wunsch nach solchen Zubereitungen, die nicht nur besonders hautfreundlich sind, sondern insbesondere ölige Anschmutzungen - im weitesten Sinne auch dekorative Kosmetik - schnell und rückstandsfrei entfernen. In vielen Fällen wird der Hersteller solcher Endzubereitungen davon absehen, die einzelnen Einsatzstoffe selbst abzumischen, er wird vielmehr versuchen auf sogenannte "Allzweck-Compounds" zurückzugreifen. Hierunter werden Abmischungen verstanden, die als Grundlage für ganz unterschiedliche Endprodukte dienen können. Diese Abmischungen werden immer häufiger als Mikroemulsionen mit vielseitigen Vorteilen angeboten. Im einfachsten Fall werden die konzentrierten Mikroemulsionen durch Zusatz von Wasser auf die Anwendungskonzentration verdünnt und stellen dann selbst die Mittel dar. In der Regel wird man sie jedoch mit entsprechenden Zusatzstoffen addieren. Je nach Anwendungsgebiet dieser "Allzweck-Compounds" in Form einer Mikroemulsion werden unterschiedliche Anforderungen gestellt, die im besten Fall bereits von der Mikroemulsion erfüllt werden. Im Einzelfall müssen weitere Komponenten zugefügt werden, die diese Anforderungen erfüllen, ohne die positiven Merkmale der Mikroemulsion zu stören. Ein besonderes Augenmerk verdient der Punkt der dermatologischen Unbedenklichkeit, da hier die Verbrauchererwartungen in den letzten Jahren gestiegen sind. Vor dem Hintergrund einer steigenden Anzahl von Konsumenten, die eine empfindliche Haut haben, werden Mittel die diesbezüglich vorteilhaften Eigenschaften aufweisen immer wichtiger.

Die WO 00/71658 schlägt vor, in Handgeschirrspülmittel ein hautfreundliches, polymeres Mittel als Schaumstabilisator einzuarbeiten, um auf diese Weise nicht Haut irritierende Reinigungsmittel herzustellen.

Die EP 0 410 567 A1 schlägt vor, Handgeschirrspülmittel durch Zugabe bestimmter hautfreundlicher Additive, ausgewählt aus der Gruppe der Kohlenwasserstoffe, Ester, Amine, Amide, quaternären Ammoniumverbindungen oder von Alkoholen hautfreundlicher zu machen. Eine ausgewählte Komponente sind Wachse, insbesondere Bienenwachs. Die Mittel gemäß der Lehre der Schrift werden hergestellt, indem man die festen Inhaltsstoffe aufschmilzt und dann bei Temperaturen von > 70 °C mit der wässerigen Phase verrührt.

Die EP 0 559 304 A2 beschreibt Handgeschirrspülmittel in Form einer Emulsion oder Mikroemulsion, die Alkylpolyglycosid, Co-Tenside und hautglättende Verbindungen enthalten können.

Im Bereich der kosmetischen Reinigungsmittel besteht ebenfalls ein Bedarf Mittel aufzufinden, die die Erwartungen der Endkunden als auch der Endprodukthersteller besser erfüllen.

Nach der Wäsche fühlen sich Haut und Haare häufig rau und spröde an, insbesondere wenn sie schon durch Umwelteinflüsse vorgeschädigt sind. Haare können überdies noch durch Färben oder Dauerwellen geschädigt sein und zeichnen sich dann nach der Haarwäsche oft durch einen trockenen strohigen Griff aus.
Ziel der kosmetischen Reinigungsmittel ist es daher, den durch tägliches Waschen verursachten Fett- und Wasserverlust bei Haut und Haaren auszugleichen. Die Körperpflegeprodukte sollen vor Umwelteinflüssen, insbesondere vor Sonne und Wind schützen und die Hautalterung verzögern.

Daher werden in Shampoo-Zusammensetzungen häufig Konditionierer eingesetzt, die diesen Nachteilen entgegen wirken sollen. Häufig findet man daher Shampoo-Zusammensetzungen, die als Konditionierer Silikone enthalten. Diese können jedoch irreversibel auf die Haare aufziehen und verursachen so ihrerseits negative Auswirkungen auf den Griff, im schlimmsten Fall sogar zu Problemen beim Färben und Dauerwellen von Haaren.

In vielen kosmetischen Zubereitungen kommen als Konditionierungsmittel Öle und Wachse in Frage. Diese sind in ihrer Wirkung jedoch bei weitem nicht so ausgeprägt wie Silikone. Diese Öle und Wachse können bisher nur in geringen Mengen in den Zubereitungen stabilisiert werden.

In der Europäischen Patentanmeldung EP 1 152 051 A2 werden wässrige Reinigungsmittel auch für die Haut offenbart, die neben Konzentraten aus Fettsäurealkylester, Emulgatoren, Rückfettungsmittel und Polyole weitere Hilfs- und Zusatzstoffe wie Ölkörper und/oder Wachse enthalten können.

In der WO2008155075 und WO2008155073 A2 werden kosmetische Zubereitungen beschrieben, die neben nicht-alkoxylierten Tensiden eine Mikroemulsion und mindestens ein kationisches Polymer enthalten. Diese Zubereitungen werden eingesetzt als Konditionierungsmittel in Shampoo und Haarbehandlungsmitteln. Die Mikroemulsion enthält APG, Glycerinmonoester, einen Ölkörper und Wasser. Zur besseren Konditionierwirkung muss zwingend ein kationisches Polymer eingesetzt werden.

Aus der Europäischen Patentanmeldung EP 0813861 A2 sind Sonnenschutzmittel in Form von O/W-Mikroemulsionen bekannt, die Ölkörper, Alkyloligoglykoside und/oder Fettsäure-N-alkylpolyhydroxyalkylamide und Lichtschutzfilter enthalten. Als weitere Inhaltsstoffe können Hilfs- und Zusatzstoffe wie anionische Tenside, Co-Emulgatoren, Wachse oder Kationpolymere enthalten sein.

Nachteilig an den Mikroemulsionen des Standes der Technik ist, dass neben den drei Hauptbestandteilen Tensid, Cotensid und Ölphase die Einarbeitung hochviskoser, wachsartiger Substanzen sehr schwierig ist.

Problematisch bei der Bereitstellung von Mikroemulsionen mit anionischen, kationischen-oder amphoteren Tensiden ist weiterhin, dass die Ölkomponenten nur sehr schwierig einzusetzen sind, da die Tenside häufig zu wasserlöslich sind und daher kaum Emulgiereigenschaften aufweisen.

In DE 3534733 werden Mikroemulsionen beschrieben, wobei jedoch der Anteil der solubilisierten Ölkomponenten im Bereich von 0,5 bis 3 Gew.% gering ist. Die Problematik der Einarbeitung größerer Ölmengen wird auch in WO 9948473 deutlich, hier wird die Einarbeitung von lediglich 0.5-1 Gew.% Öl angeführt.

Es ist bekannt, dass Öl-in-Wasser-Emulsionen, die mit nichtionischen Emulgatoren hergestellt sind, beim Erwärmen häufig eine Phaseninversion erleiden, d. h., dass bei höheren Temperaturen die äußere, wässerige Phase zur inneren Phase werden kann. Dieser Vorgang ist in der Regel reversibel, so dass sich beim Abkühlen wieder der ursprüngliche Emulsionstyp zurückbildet. Emulsionen, die oberhalb der Phaseninversionstemperatur hergestellt wurden, weisen im Allgemeinen eine niedrige Viskosität und hohe Lagerstabilität auf.

So beschreibt die WO 98/40044 wässrige Zubereitungen wasserlöslicher Tenside, die Lipid-Tensid-Mischmizellen mit einer mittleren Teilchengröße von unter 500 nm aufweisen und dabei weiß-bläulich erscheinen. Gegenstand der WO 98/15255 sind Mikroemulsionsgele vom Typ Öl-in-Wasser, bei denen die Öltröpfchen in der Wasserphase durch assoziative Verdicker stabilisiert werden.

Die Aufgabe der vorliegenden Erfindung hat somit darin bestanden, hautfreundliche kosmetische Reinigungsmittel bereit zu stellen, die die geforderten Aufgaben besser erfüllen als bekannte Produkte, deren Reinigungsverhalten dem Stand der Technik entspricht oder bestenfalls diese sogar noch übertrifft und die nach der Anwendung auf der Haut ein angenehmes Hautgefühl hinterlassen. Auf der Haut sollte ein Gefühl einer weichen, reichhaltig versorgten Haut entstehen und eventuell gereinigte Haare sollten einen angenehmen Griff erhalten ohne fettendes Gefühl zu hinterlassen.

Eine weitere Aufgabe der vorliegenden Erfindung ist es, ölhaltige Reinigungsprodukte bereit zu stellen, die sensorisch angenehm auf der Haut aufgetragen, verteilt und abgewaschen werden können, wobei die hautpflegende Ölphase auf der Haut verbleibt. Desweiteren sollten die Mittel ein nachhaltig pflegendes und angenehmes Hautgefühl hinterlassen ohne die reinigende Wirkung beim Spülvorgang zu verlieren. Das Reinigungsmittel sollte Mikroemulsionen enthalten, die gut formulierbar sind, stabil über längeren Zeitraum und die Eigenschaft ein angenehmes Hautgefühl zu vermitteln bereits beinhalten. Außerdem sollten kosmetische Haarpflegemittel bereit gestellt werden, deren Konditionierungsleistung der von silikonhaltigen Zubereitungen entspricht oder bestenfalls diese sogar noch übertrifft.

Es war für den Fachmann nicht vorauszusehen, dass die Aufgabe gelöst werden konnte durch ein kosmetisches Reinigungsmittel, das diese Mikroemulsion enthält, welche sich durch die Mischung von Tensid, Cotensid, und einer Mischung aus organischer Ölphase und Wachsen auszeichnet.

Dank der Mikroemulsion liegt diese Reinigungsmittelkomponente als dünnflüssiges Mittel vor und kann sehr gut in jegliche Art der Reinigungs- und Pflegemittel eingearbeitet werden, so dass transparente oder leicht trübe Produkte herstellbar werden. Bevorzugt ist der Einsatz als kosmetisches Reinigungsmittel in Duschgel-Formulierungen oder Shampoo-Zubereitungen. Naheliegend ist dadurch auch der bevorzugte Einsatz in Kombinationsformulierungen für Haut und Haar (two in one - Produkte). Es konnte gezeigt werden, dass kosmetisches Reinigungsmittel mit einer Mikroemulsion enthaltend eine Wachskomponente und als Ölphase mindestens ein Esteröl hervorragende Eigenschaften in der Körperpflege zeigt, weil die Kombination dieser beiden Ölkörper dazu führt, dass ein angenehmes nachhaltig pflegendes Hautgefühl zu spüren ist. Die beiden Ölkörper führen in ihrer Kombination zu einer langanhaltenden Anhaftung an Haut und Haaren ohne jedoch fettig zu wirken bzw. ein unangenehm fettendes Gefühl zu hinterlassen, da sie in der Mikroemulsion eingearbeitet vorliegen. Dieser Effekt konnte durch Depositionstests nachgewiesen werden. Für eine Duschgelformulierung ist es weiterhin von großem Vorteil, dass eine auf Esteröle basierte wachshaltige Mikroemulsion in Kombination mit kationischen Polymeren das Hautgefühl nach dem Duschvorgang signifikant verbessert. Dies konnte in sensorischen Tests belegt werden.

Ein weiterer Vorteil der erfindungsgemäß enthaltenen Mikroemulsion liegt darin, dass sie bevorzugt frei von alkoxylierten Verbindungen ist und somit auch in Kosmetikprodukten eingearbeitet werden kann, bei denen der Verbraucher auf die "grünen Kosmetika" gesteigerten Wert legt.

Ein erster Gegenstand der vorliegenden Anmeldung ist gerichtet auf kosmetische Reinigungsmittel enthaltend
A) eine Mikroemulsion, enthaltend
   (a) mindestens ein Alkyl(oligo)glycosid,
   (b) mindestens ein von (a) unterschiedliches Co-Tensid,
   (c) mindestens eine nicht-wasserlösliche organische Ölkomponente,
   (d) mindestens ein Wachs und
   (e) Wasser
B anionische Tenside
C kationische Polymere
D gegebenenfalls weitere Tenside
E gegebenenfalls kosmetische Zusatzstoffe
F Wasser.

Daneben können die Mikroemulsionen A) als zusätzliche Komponente (f) eine kationische Verbindung, insbesondere quaternäre Ammoniumverbindungen oder ein kationisches Polymer enthalten. Unter quaternären Ammoniumverbindungen werden hierbei insbesondere quaternierte Fettsäuretriethanolaminestersalze verstanden. Geeignet sind aber ebenso Alkylammoniumhalogenide.

Weiterhin sind noch optional weitere Inhaltsstoffe möglich, wie Biozide, Konservierungsmittel, pH-Regulantien, Farbstoffe, Entschäumer, oder Parfüme als Komponente (g). Bevorzugt bei der Herstellung von kosmetischen Reinigungsmitteln sind kosmetische Zusatzstoffe die später im Text noch beschrieben werden.

Für die Verwendung in kosmetischen Reinigungsmitteln wie Shampoo, Babypflegeprodukten, Schaumbad, Badeöl kann die Mikroemulsion direkt eingesetzt werden oder mit üblichen Zusatzstoffen wie Schaumbildner, Verdickern, kationischen Polymeren, Konservierungsmitteln, Wirkstoffen formuliert werden. Für den Einsatz als Duschgel ist der Zusatz von Schaumbildnern und Verdickern bevorzugt, um eine gelartige Konsistenz zu erhalten. Für den Einsatz als Tränkungsmittel für Flächengebilde ist eine Verdünnung mit Wasser bevorzugt.

In einer bevorzugten Ausführungsform ist eine Mikroemulsion A) enthalten, enthaltend:
a) 1 - 35 Gew.-% mindestens eines Alkyl(oligo)glycosides
b) 1 - 30 Gew.-% mindestens eines Cotensides
c) 5 - 60 Gew.-% einer organischen Ölphase
d) 0,5 - 15 Gew.-% mindestens einer Wachskomponente
e) Wasser add 100 Gew.-%
f) 0 - 10 Gew.-% kationische Polymere
g) 0 - 10 Gew.-% weitere Inhaltsstoffe .

In einer besonders bevorzugten Ausführungsform ist eine Mikroemulsion im kosmetischen Reinigungsmittel enthalten, enthaltend:
a) 10 - 25 Gew.-% mindestens eines Alkyl(oligo)glycosides
b) 10 - 20 Gew.-% mindestens eines Cotensides
c) 10 - 50 Gew.-% einer organischen Ölphase enthaltend Esteröle
d) 0,5 - 10 Gew.-% mindestens einer Wachskomponente
e) Wasser add 100 Gew.-%
g) 0 - 10 Gew.-% kosmetische Zusatzstoffe

Insbesondere bevorzugt besteht die Mikroemulsion, in den kosmetischen Reinigungsmitteln aus a) bis g) in den im oberen Abschnitt genannten Mengenverhältnissen.

Erfindungsgemäß liegen die Teilchen in der Mikroemulsion oder in den kosmetischen Reinigungs- und Pflegemittel feinteilig vor. Im Sinne der Erfindung bedeutet **"feinteilig"** eine mittlere Teilchengröße, speziell 3 bis 100 nm für die Teilchen in der Mikroemulsion und bei Verdünnung der Mikroemulsion eine Teilchengröße ≤ 5000 nm. Die Teilchengröße wird bestimmt nach der DLS Methode mit einem Gerät der Bezeichnung Horiba LB-500.

### Mikroemulsion

Mikroemulsionen sind an sich bekannt. Mikroemulsionen sind makroskopisch homogene, optisch transparente, niedrigviskose, thermodynamisch stabile Mischungen. Je nach verwendetem Tensidtyp zeigt die Mikroemulsion ein temperaturabhängiges Phasenverhalten. Vor allem manche nichtionischen Tenside, und hier vor allem Tenside, deren hydrophile Molekülteile aus Ethoxy- oder Propoxy-Gruppen aufgebaut sind, führen zu einem charakteristischen temperaturabhängigen Phasenverhalten bei Mikroemulsionen.

Voraussetzung für die Bildung von Mikroemulsionen ist eine extrem niedrige Grenzflächenspannung zwischen der wasser- und der ölreichen Phase. Diese kann bei den Mikroemulsionen Werte zwischen 10⁻¹ und 10⁻⁵ mNm⁻¹ annehmen.

Die mittleren Teilchengrößen der Mikroemulsionen liegen üblicherweise unter 100 nm, vorzugsweise zwischen 3 und 100 nm. Sie weisen eine hohe Transparenz auf und sind beim Zentrifugieren bei 2000 UPM für mindestens 30 Minuten gegenüber einer sichtbaren Phasenseparation stabil. Die Mikroemulsionen im Sinne der vorliegenden Lehre zeigen vorzugsweise eine mittlere Teilchengröße von weniger als 100 nm. Die Leitfähigkeit der erfindungsgemäßen Mikroemulsionen liegt vorzugsweise im Bereich von größer/gleich 500 µSi/cm und besonders bevorzugt bei größer/gleich 1000 µSi/cm. Ein bevorzugter Bereich liegt bei 800 bis 1500 µSi/cm. Die erfindungsgemäßen Mikroemulsionen sind vorzugsweise transparent, insbesondere zeigen sie eine Transparenz von größer/gleich 80 % bei 40 °C, wobei Transparenz-Werte von größer als 90 % bei 40 °C typisch sind. Bevorzugt sind solche Mikroemulsionen, die eine Transparenz, gemessen bei 40 °C von 95 bis 100 % aufweisen.

Die Herstellung der Mikroemulsionen erfolgt vorzugsweise einfach durch Vermischen der Ölphase mit den weiteren öllöslichen Inhaltsstoffen, Erwärmen der Ölphase über den Schmelzpunkt aller Bestandteile und anschließender Zugabe der wässrigen tensidhaltigen Phase. Alternativ kann auch die erwärmte Ölphase in die wässrige Phase gegeben werden. Die thermodynamisch stabile Mikroemulsion bildet sich dann spontan, ggf. muss noch etwas gerührt werden.

Die Mikroemulsionen gemäß der vorliegenden Lehre sind vorzugsweise vom Typ Öl-in-Wasser (O/W) oder haben eine bikontinuierliche Struktur. Die Mikroemulsionen enthalten als äußere Phase somit vorzugsweise Wasser, und weiterhin Alkyl(oligo)glycoside und eine davon unterschiedliches Co-Tensid, sowie eine Ölkomponente und ein Wachs. Die Mirkoemulsionen können aber auch als Wasser-in-Öl Emulsion vorliegen.

### Komponente (a)

Bevorzugt werden als Komponente (a) der vorliegenden Erfindung Mikroemulsionen auf Basis von Alkyl(oligo)glykosiden eingesetzt.
Alkyl- und Alkenyloligoglykoside stellen bekannte nichtionische Tenside dar, die der allgemeinen Formel (I) folgen,

R¹O-[G]ₚ (I)

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Die Mikroemulsionen im Sinne der vorliegenden Lehre enthalten die Komponente (a) vorzugsweise in Mengen von 1 bis 35 Gew.-%, bezogen auf das Gesamtgewicht der Mikroemulsion. In den kosmetischen Reinigungsmitteln sind Mengen von 10 bis 30 Gew.-%, besonders bevorzugt von 15 bis 25 Gew.-% bezogen auf das Gesamtgewicht der Mikroemulsion enthalten.

### Komponente (b) Cotensid

Als weitere zwingende Komponente enthalten die Mikroemulsionen mindestens ein Co-Tensid, das strukturell von der Komponente (a) verschieden sein muss und ein Polyolfettsäureester darstellt. Im Sinne der Erfindung umfassen Polyole deren Fettsäurester als Cotenside eingesetzt werden, Alkohole mit mindestens 3 Kohlenstoffatomen und mindestens drei Hydroxylgruppen.

Für die Herstellung von kosmetischen Reinigungsmitteln werden als Cotenside Fettsäureester von Polyolen bevorzugt, die ausgewählt sind aus der Gruppe, die gebildet wird von Zuckerester, W/O Emulgatoren wie Sorbitanester, Sorbitolpartialester, Polysorbate, Polyglycerylester, Polyglycerylpartialester, speziell beispielsweise Polyglyceryl-2-dipolyhydroxystearat, Polyglyceryl-3-diisostearat. Weiterhin bevorzugt als Cotenside für die Herstellung von kosmetischen Reinigungsmitteln sind ein und zweiwertige Alkohole von linearen oder verzweigten Alkanen.

Für die Herstellung von kosmetischen Reinigungsmitteln sind die Cotenside vorzugsweise in Mengen von 10 - 20 Gew.-%, besonders bevorzugt in Mengen von 4 - 20 Gew.-% bezogen auf das Gesamtgewicht der Mikroemulsion enthalten.

Die Co-Tenside können auch als Mischung eingesetzt werden.

### Komponente (c) Organische Ölphase

Die Mikroemulsionen im Sinne der vorliegenden Lehre enthalten als weiteren obligatorischen Bestandteil eine wasserunlösliche so genannte Ölphase, die mindestens eine Ölkomponente enthält, also eine nicht-wasserlösliche organische Phase. Vorzugsweise in Mengen von 5 bis 60 Gew.-% bezogen auf das Gesamtgewicht der Mikroemulsion.

Bevorzugt enthalten die Mikroemulsionen wasserunlösliche Ölkomponenten oder Ölphasen ausgewählt aus der Gruppe von Guerbetalkoholen auf Basis von Fettalkoholen mit 6 bis 18 C-Atomen, Estern linearer C₆-C₂₂-Fettsäuren mit linearen oder verzweigten C₆-C₂₂-Fettalkoholen bzw. Estern von verzweigten C₆-C₁₃-Carbonsäuren mit linearen oder verzweigten C₆-C₂₂-Fettalkoholen, Ester von linearen C₆-C₂₂-Fettsäuren mit verzweigten Alkoholen, Estern von C₆-C₂₂-Fettalkoholen und/oder Guerbetalkoholen mit aromatischen Carbonsäuren, Triglyceriden auf Basis C₆-C₁₀-Fettsäuren, Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Öle, verzweigten primäre Alkoholen, substituierten Cyclohexanen, linearen und verzweigten C₆-C₂₂-Fettalkoholcarbonaten, Guerbetcarbonate auf Basis von Fettalkoholen mit 6 bis 18, vorzugsweise 8 bis 10 C Atomen, Estern der Benzoesäure mit linearen und/oder verzweigten C₆-C₂₂-Alkoholen, linearen oder verzweigten, symmetrischen oder unsymmetrischen Dialkylethern mit 6 bis 22 Kohlenstoffatomen pro Alkylgruppe, aliphatische bzw. naphthenische Kohlenwasserstoffe und Dialkylcyclohexanen.

Die Mikroemulsion für die kosmetischen Reinigungsmittel enthält wasserunlösliche Ölkomponenten vorzugweise in Mengen von 5 - 50 Gew.-%, bevorzugt 10 bis 50 Gew.-% Aktivsubstanz bezogen auf das Gesamtgewicht der Mikroemulsion. Für kosmetische Reinigungsmittel sind bevorzugte organische Ölphasen, ausgenommen alkoxylierte Verbindungen, flüssige Esteröle also Ester linearer C₆-C₂₂-Fettsäuren mit linearen oder verzweigten C₆-C₂₂-Fettalkoholen bzw. Estern von verzweigten C₆-C₁₃-Carbonsäuren mit linearen oder verzweigten C₆-C₂₂-Fettalkoholen, Ester von linearen C₆-C₂₂-Fettsäuren mit verzweigten Alkoholen, Ester von C₁₈-C₃₈-Alkylhydroxycarbonsäuren mit linearen oder verzweigten C₆-C₂₂-Fettalkoholen, Ester von linearen und/oder verzweigten Fettsäuren mit mehrwertigen Alkoholen und/oder Guerbetalkoholen, Estern von C₆-C₂₂-Fettalkoholen und/oder Guerbetalkoholen mit aromatischen Carbonsäuren, wobei die Ester aus Fettsäuren mit mehr als 14 C-Atomen und Alkoholen mit mehr als 14 C-Atomen erfindungsgemäß zu den Wachsen zählen, sobald diese bei 21 °C fest sind. Desweiteren Triglyceride auf Basis C₆-C₁₀-Fettsäuren, Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Öle, lineare und verzweigte C₆-C₂₂-Fettalkoholcarbonate. Insbesondere bevorzugt sind die Esteröle ausgewählt aus der Gruppe, die gebildet wird von Isopropylpalmitat, Isopropylmyristat, Ethylhexylpalmitat, Ethylhexylstearate, Di-n-Octylcarbonate, Caprylylcaprylat, Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat, Dioctyl Malate, Propylenglycol, Dimerdiol oder Trimertriol oder deren Mischungen. Aus diesen Esterölen sind besonders bevorzugt Caprylylcaprylat, Cococaprylat und Dialkylcarbonate.

Besonders bevorzugt besteht die organische Ölphase für den Fall der kosmetischen Reinigungsmittel aus flüssigen Esterölen.

Neben den Ölen kommen auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage. Tocopherole und ätherische Öle eignen sich ebenfalls als Ölkomponente.

Als Kohlenwasserstoffe werden organische Verbindungen bezeichnet, die nur aus Kohlenstoff und Wasserstoff bestehen. Sie umfassen sowohl cyclische als auch acyclische (=aliphatische) Verbindungen. Sie umfassen sowohl gesättigte wie einfach oder mehrfach ungesättigte Verbindungen. Die Kohlenwasserstoffe können linear oder verzweigt sein. Je nach Anzahl der Kohlenstoffatome im Kohlenwasserstoff kann man die Kohlenwasserstoffe einteilen in ungradzahlige Kohlenwasserstoffe (wie beispielsweise Nonan, Undecan, Tridecan) oder geradzahlige Kohlenwasserstoffe (wie beispielsweise Octan, Dodecan, Tetradecan). Je nach Art der Verzweigung kann man die Kohlenwasserstoffe einteilen in lineare (= unverzweigte) oder verzweigte Kohlenwasserstoffe. Gesättigte, aliphatische Kohlenwasserstoffe werden auch als Paraffine bezeichnet.

### Komponente (d) - Wachse

Als weiteren Bestandteil enthalten die Mikroemulsionen Wachse. Diese können auch in Mischung mit den im vorherigen Abschnitt genannten Ölen vorliegen. Im Sinne der Erfindung stellen Wachse natürliche Substanzen tierischer oder pflanzlicher Herkunft dar, welche bei Raumtemperatur (21 °C) fest sind jedoch im Allgemeinen eine gewisse Verformbarkeit besitzen. Wachse sind in Wasser unlöslich, jedoch in Ölen löslich und zur Bildung von wasserabweisenden Filmen befähigt.

Typische Beispiele für Wachse im Sinne der vorliegenden Lehre sind natürliche Wachse, wie z.B. Shorea Stenoptera Butter (cegesoft SH), Sheabutter, Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse. Weiterhin fallen Ester langkettiger Fettsäuren (mindestens 14 C-Atome) mit langkettigen Fettalkoholen (mindestens 14 C-Atome) wie Myristylmyristat (Cetiol MM) oder C₁₆-C₁₈ Triglyceride von Olus Öl (Cegesoft PS6) unter den Begriff der Wachse.

Für die Verwendung in kosmetischen Reinigungsmitteln sind die natürlichen Wachse besonders bevorzugt und unter denen ist die Sheabutter insbesondere bevorzugt. Sheabutter (auch Sheafett, Karitefett oder Caritefett, Galambutter) ist ein natürlicher fester Fettstoff, der aus der Pflanze Butyrospermum parkii, dem afrikanischen Sheabutterbaum, gewonnen wird und in kommerziellen Mengen verfügbar ist. Ihr Schmelzbereich liegt bei 35 bis 42 °C. Üblicherweise enthält Sheabutter 89 bis 98 Gew.-% Triglyceride, Glycerinpartialester und freie Fettsäuren sowie einen Gehalt von 2 bis 11 Gew.-% unverseifbarer Anteile, von denen Kohlenwasserstoffe ("Karitene"), Triterpenalkohole und Sterole die wichtigsten sind.

### Komponente (e) Wasser

Ein weiterer wesentlicher Bestandteil der Mikroemulsionen und des kosmetischen Mittels ist Wasser. Das Wasser sollte vorzugsweise demineralisiert sein. Die Mikroemulsionen enthalten vorzugsweise bis zu 90 Gew.-% Wasser.

Bevorzugte Bereiche für den Wasseranteil in der Mikroemulsion für die Verwendung zur Herstellung von kosmetischen Reinigungsmitteln sind Mengen von 5 bis 60, besonders von 5 bis 50 Gew.-% und insbesondere von 10 bis 40 Gew.-% Wasser in der Mikroemulsion. Für die kosmetischen Reinigungsmittel ergibt sich ein bevorzugter Wasseranteil von größer 80 Gew.-% bezogen auf die Gesamtmenge der kosmetischen Reinigungsmittel. Dies bedeutet, dass in den 80 Gew.-% der Anteil an Wasser aus der enthaltenden Mikroemulsion einbezogen ist. Ebenso ist Wasser aus den anderen Inhaltstoffen, die nie frei von Wasser sind, mit einbezogen.

### Weitere Inhaltsstoffe

Daneben können die Mikroemulsionen als zusätzliche Komponente (f) eine kationische Verbindung, insbesondere quaternäre Ammoniumverbindungen oder ein kationisches Polymer enthalten. Unter quaternären Ammoniumverbindungen werden hierbei insbesondere quaternierte Fettsäuretriethanolaminestersalze verstanden. Geeignet sind aber ebenso Alkylammoniumhalogenide.

Geeignete kationische Polymere sind beispielsweise kationische Cellulosederivate, wie z.B. eine quaternierte Hydroxyethylcellulose, kationische Stärke, Copolymere von Diallylammoniumsalzen und Acrylamiden, quaternierte Vinylpyrrolidon/Vinyl-imidazol-Polymere, wie z.B. Luviquat (BASF), Kondensationsprodukte von Polyglycolen und Aminen, quaternierte Kollagenpolypeptide, wie beispielsweise Lauryldimonium hydroxypropyl hydrolyzed collagen (Lamequat), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine), Copolymere der Acrylsäure mit Dimethyldiallyl-ammoniumchlorid (Merquat 550), Polyaminopolyamide, wie z.B. beschrieben in der FR-A 2252840 sowie deren vernetzte wasserlöslichen Polymere, kationische Chitinderivate, wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, quaternierte Ammoniumsalz-Polymere, wie z.B. Mirapol A-15, Mirapol AD-1, Mirapol AZ-1 der Firma Miranol.

Weitere bevorzugte kationische Polymere sind gewählt aus der Gruppe der Homo- oder Copolymere von Ester- oder Amidderivaten der Acryl- oder Methacrylsäure (z.B. INCI: Polyquaternium-7, oder PQ-7), Homopolymeren aus Methacryloylethyltrimethylammonium Chlorid (INCI: Polyquaternium-37, oder PQ-37), quaternären Copolymeren aus Hydroxyethylcellulose und Diallyl-dimethyl-ammoniumchlorid (INCI: Polyquaternium-4, oder PQ-4), polymeren quaternisierten Ammoniumsalzen von Hydroxyethylcellulose, welche mit einem trimethylammonium-substituierten Epoxid modifiziert sind (INCI: Polyquaternium-10, oder PQ-10), depolymerisierten Guar Gum Derivaten, welche quaternisiert sind (INCI: Guar Hydroxypropyl Trimonium Chlorid) oder quaternisierte Guarderivate und quaternären Copolymeren aus Hydroxyethylcellulose und Diallyl-demethylammoniumchlorid. In einer bevorzugten Ausführungsform wird das kationische Polymer ausgewählt aus der Gruppe, die gebildet wird von Polyquaternium-7, Polyquaternium-10 und kationischen Guar-Derivaten.

Weiterhin können mit Vorteil kationische Polymere gemäß der Lehre der EP 1 767 554 A1 Verwendung finden, die seitens der Anmelderin unter der Marke Polyquart Pro vertrieben werden. Die erfindungsgemäßen Mikroemulsionen enthalten vorzugsweise 0,05 bis 2 Gew.-% dieser kationischen Polymere.

Die erfindungsgemäßen kosmetischen Reinigungsmittel enthalten als Komponente C) die genannten kationischen Polymere. Auch hier sind die bevorzugten kationischen Polymere ausgewählt aus der Gruppe, die gebildet wird von Polyquaternium-7, Polyquaternium-10 und kationischen Guar-Derivaten. In einer besonders bevorzugten Ausführungsform werden kationische Guar-Derivate bevorzugt, da diese dem "grünen" Konzept entsprechen.

### Komponente (g) - weitere Inhaltsstoffe

Weiterhin sind noch optional weitere Inhaltsstoffe für die Mikroemulsion möglich, die ausgewählt sind aus der Gruppe, die gebildet wird von Hydrotope wie Glycerin, Konservierungsmittel, Zitronensäure, Phenoxyethanol, UV-Lichtschutzfilter, Antioxidantien, biogene Wirkstoffe, Parfum, Farbstoffe, Biozide, Entschäumer, und pH-Regulantien.

Diese optionalen Komponenten sind in Summe vorzugsweise in Mengen von 1 bis 30 Gew.-% und insbesondere in Mengen von 2 bis 15 Gew.-% in den Mikroemulsionen enthalten, jeweils bezogen auf das Gesamtgewicht der Mikroemulsion.

Die genannten weiteren Inhaltsstoffe für die erfindungsgemäß verwendete Mikroemulsion können im Sinne der Erfindung auch als Komponente E) in kosmetischen Reinigungsmitteln enthalten sein. Neben den genannten weiteren Inhaltsstoffen kann die Mikroemulsion und/oder die kosmetischen Reinigungsmittel, welche die Mikroemulsion enthalten, noch weitere übliche kosmetische Hilfs- und Zusatzstoffe enthalten, die dem Fachmann bekannt sind wie beispielsweise milde Tenside, Emulgatoren, Perlglanzwachse, Stabilisatoren, Salz, Verdickungsmittel, Konsistenzgeber, Selbstbräuner, Pigmente, Antioxidationsmittel, Antischuppenmittel, Filmbildner, Quellmittel, Insektenrepellentien, Deodorant- und Antitranspirantwirkstoffe, biogene Wirkstoffe. Als biogene Wirkstoffe sind dabei insbesondere Tocopherol, Tocopherolacetat, Tocopherolpalmitat, Desoxyribonucleinsäure, Coenzym Q10, Ascorbinsäure, Retinol- und Retinylderivate, Bisabolol, Allantoin, Phytantriol, Panthenol, AHA-Säuren, Aminosäuren, Ceramide, essentielle Öle, Hyaloronsäure, Creatin, Proteinhydrolysate, Pflanzenextrakte, Peptide und Vitaminkomplexe bevorzugt.

In einer weiteren bevorzugten Ausführungsform der Erfindung enthält die Mikroemulsion (alle Angaben bezogen auf Aktivsubstanz (AS)):
4 - 25 Gew.-% der Komponente (a)
4 - 20 Gew.-% der Komponente (b)
5 - 50 Gew.-% der Komponente (c)
0,5-15 Gew.-% der Komponente (d)
add 100 Gew.-% der Komponente (e)
0 - 10 Gew.-% der Komponente (f)
0 - 5 Gew.-% anderer Inhaltsstoffe (g)
mit der Maßgabe, dass die Summe aus (a) bis (g) 100 ergibt.

Die wässerigen Mikroemulsionen gemäß der vorliegenden Beschreibung weisen vorzugsweise eine pH-Wert zwischen 2 und 9, wobei die Bereiche von 3 bis 8 vorteilhaft sein können.

### Herstellung der Mikroemulsionen

Hergestellt werden diese Emulsionen beispielsweise indem man zunächst in einem ersten Schritt eine Mikroemulsion herstellt, enthaltend vorzugsweise mindestens 10 - 20 Gew.-% eines Alkyl(oligo)glycosids der allgemeinen Formel R¹O-[G]ₚ in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht und vorzugsweise 1 - 30 Gew.-% eines Cotensides und vorzugsweise 5 - 50 Gew.-% eines Ölkörpers und den Rest auf 100 Gew.-% Wasser zusammengibt und diese Mischung, ggf. unter Erwärmung auf Temperaturen von 30 bis 80 °C verrührt.

In einer bevorzugten Ausführungsform werden die Mikroemulsionen nach der Lehre der WO 08/155075 A1 hergestellt: Das dort offenbarte Verfahren ist ein zweistufiger Prozess, bei dem im ersten Schritt auf an sich bekannte Weise eine Mikroemulsion hergestellt wird. Die Herstellung der Mikroemulsionen in Schritt 1 erfolgt vorzugsweise durch Vermischen der Ölphase mit den weiteren öllöslichen Inhaltsstoffen, Erwärmen der Ölphase über den Schmelzpunkt aller Bestandteile und anschließender Zugabe der wässrigen tensidhaltigen Phase. Die thermodynamisch stabile Mikroemulsion bildet sich dann spontan, ggf. muss noch etwas gerührt werden.

Die Mikroemulsionen werden erfindungsgemäß zu an sich bekannten Reinigungsmitteln hinzu gegeben, um bei diesen die Hautfreundlichkeit zu verbessern. Bevorzugt werden die Mikroemulsionen kosmetischen Reinigungsmitteln, insbesondere Duschgelen zugesetzt.

Dazu ist es vorteilhaft, wenn die Mikroemulsion bei Temperaturen von 35 bis 65 °C, vorzugsweise von 40 bis 50 °C mit den anderen Bestandteilen des Reinigungsmittels vermischt wird. Wegen des Anteils an der wasserunlöslichen Ölphase, bzw. der Wachskomponente kann es bei niedrigeren Verarbeitungstemperaturen zu Trübungen der Emulsion kommen.

Die Mikroemulsion wird im Sinne der Erfindung mit weiteren Zusatzstoffen als kosmetisches Reinigungsmittel eingesetzt.

Folgerichtig enthält das kosmetische Reinigungsmittel des Weiteren
B) anionische Tenside bevorzugt alkoxylierte wie beispielsweise Sodium laurylether sulfate,
C) kationische Polymere,
D) gegebenenfalls weitere Tenside
E) gegebenenfalls weitere kosmetische Zusatzstoffe und
F) Wasser.

Die erfindungsgemäß hergestellten kosmetischen Reinigungsmittel in Form feinteiliger Emulsionen können zur Herstellung von kosmetischen Reinigungsmitteln verwendet werden, wie beispielsweise Duschgele, Duschbäder, Haarshampoos, Haarlotionen, Schaumbäder, Handwaschmitteln, Gesichtsreinigern, make-up-Entfernern, Badezubereitungen, Babypflegeprodukten, Cremes, Gele, Lotionen, alkoholische und wässerig/alkoholische Lösungen, Emulsionen, Wachs/ Fett-Massen, Stiftpräparaten, Pudern oder Salben.

Bevorzugt sind dünnflüssige, transparente bis leicht trübe Gele zur Reinigung von Haut oder Haaren.

Die kosmetischen Reinigungsmittel können als Tränkungsmedium für Tücher, Gewebe dienen, die nass oder trocken vom Verbraucher angewendet werden oder auch aus einem Pumpfoamer appliziert werden.

Die Herstellung der kosmetischen Reinigungsmittel erfolgt in an sich bekannter Art und Weise. Die Mikroemulsionen gemäß der obigen Beschreibung werden den Inhaltsstoffen der Reinigungsmittel hinzugefügt, wobei die Wirkung erfindungsgemäß nur dann auftritt, wenn die fertige Mikroemulsion mit allen bestimmungsgemäßen Inhaltsstoffen mit den restlichen Inhaltstoffen des Reinigungsmittels zusammen gebracht wird.

Die Zugabe einzelner Bestandteile führt nicht zum gewünschten Ergebnis; Wesentlich ist hier das Merkmal der Mikroemulsion, um die vorliegende Lehre zu verwirklichen.

Zur Herstellung von klaren Produkten ist es weiterhin bevorzugt, dass die Zugabe der Mikroemulsion zu den weiteren Bestandteilen des Reinigungsmittels bei leicht erhöhten Temperaturen, vorzugsweise bei 30 bis 60 °C vorgenommen wird, um eine vollständige Durchmischung zu erreichen.

In einer bevorzugten Ausführungsform enthält das erfindungsgemäße kosmetische Reinigungsmittel
A 0,1 bis 20 Gew.-% der erfindungsgemäß zu verwendenden Mikroemulsion
B 5 - 20 Gew.-% anionische Tenside
C 0,02 - 2 Gew.-% kationische Polymere
D 0 - 15 Gew.-% weitere Tenside
E 0 - 10 Gew.% kosmetische Zusatzstoffe
F Wasser add 100 %.

Besonders bevorzugt ist ein kosmetisches Reinigungsmittel enthaltend
A 0,2 - 10 Gew.% der erfindungsgemäß zu verwendenden Mikroemulsion
B 8 - 15 Gew.-% anionische Tenside
C 0,05 - 1 Gew.-% kationische Polymere
D 0 - 5 Gew.% weitere Tenside
E 0 - 10 Gew.% kosmetische Zusatzstoffe
F Wasser add 100 %.

Die erfindungsgemäßen kosmetischen Reinigungsmitteln enthalten wie oben beschrieben neben der erfindungsgemäß zu verwendenden Mikroemulsion auch weitere Komponenten wie B) die anionischen Tenside die ausgewählt sind aus der Gruppe, die gebildet wird von Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid-(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate; besonders bevorzugt sind dabei die Fettalkoholethersulfate wie beispielsweise Sodiumlaurylethersulfat oder andere Verbindungen mit vergleichbarem Schaumverhalten für die Anwendung als reinigende Pflegemittel wie Duschgel, Shampoo, Handwaschmittel oder ähnliches.

### Kationische Polymere C)

Als Komponente C) enthalten die kosmetischen Mittel der vorliegenden Patentanmeldung die oben beschriebenen kationischen Polymere.

### Weitere Tenside D)

Als Komponenten D) enthalten die kosmetischen Mittel der vorliegenden Patentanmeldung weitere Tenside. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze.

Im Sinne der Erfindung bevorzugt als weitere Tenside sind amphotere bzw. zwitterionische Tenside. Typische Beispiele für amphotere bzw. zwitterionische Tenside sind Alkylbetaine, Alkylamidobetaine, Aminopropionate, Aminoglycinate, Imidazoliniumbetaine und Sulfobetaine, besonders bevorzugt ist Cocamidopropylbetain. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisothionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine wie Cocamidopropylbetain, Amphoacetate wie Sodiumcocoamphoacetat und/oder Proteinfettsäurekondensate, letztere vorzugsweise auf Basis von Weizenproteinen.

### Kosmetische Zusatzstoffe E)

Die weitere Komponenten E) der kosmetischen Zusatzstoffe ist bereits weiter oben beschrieben worden. Es ist im Sinne der Erfindung bevorzugt, dass die beschriebenen kosmetischen Zusatzstoffe in dem kosmetischen Reinigungsmittel enthalten sind und nicht in die Mikroemulsion eingearbeitet werden. Wird die Mikroemulsion jedoch ohne weitere Komponenten (B bis E) eingesetzt, kann sie diese Zusatzstoffe enthalten. Für den Einsatz als reinigendes Gel oder als Paste und Salbe sind Konsistenzgeber und Verdickungsmittel als kosmetische Zusatzstoffe bevorzugt enthalten. Diese können ausgewählt sein aus folgenden Verbindungen:

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und - diester von Fettsäuren, Polyacrylate und hydrophob modifizierte Polyacrylate, Polyacrylamide, Polymere, Polyvinylalkohol und Polyvinylpyrrolidon. Als besonders wirkungsvoll haben sich auch Bentonite erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Falls Konservierungsmittel erfindungsgemäß eingesetzt werden, sind diese bevorzugt ausgewählt aus der Gruppe, die gebildet wird von Benzoesäure und deren Salze, Zitronensäure und deren Salze, Phenoxyethanol, Benzylalkohol, Alkylparabenen, bevorzugt Ethyl-, Methyl- und Propylparaben. Als Konservierungsmittel eignen sich weiterhin beispielsweise Formaldehydlösung, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Durch Einarbeitung einer Mikroemulsion als Komponente A) in die erfindungsgemäßen kosmetischen Reinigungsmittel gelingt die transparente bis leicht trübe Einarbeitung größerer Mengen von Ölkörpern, die dann mit den kationischen Polymeren der Komponente C) in der Zusammensetzung stabilisiert durch die Tenside der Komponente B) die hervorragenden konditionierenden Eigenschaften der Zubereitung bewirken.

Für kosmetische Reinigungsmittel ist ein pH-Bereich 4 - 8 bevorzugt.

Die kosmetischen Reinigungsmittel zeigen verbesserte sensorische Eigenschaften, insbesondere bei Duschgel und Haarshampoo.

Unter sensorischen Eigenschaften werden die Eigenschaften von Mitteln verstanden, die zu einer Veränderung der Sinneswahrnehmung durch Menschen führen können, vorliegend ist damit insbesondere das Hautgefühl gemeint, dass durch direkten Kontakt der menschlichen Haut mit einem Stoff oder eine Stoffmischung ausgelöst wird. In der Praxis wird dieses Hautgefühl z.B. durch Panel-Test an Probanden ermittelt, die ihre Sinneseindrücke in Bezug auf bestimmte Parameter, wie "Trockenheit der Haut", "Weichheit der Haut" etc. durch Benotungen qualifizieren.

Die erfindungsgemäßen, Mikroemulsionen enthaltenden Reinigungsmittel verbessern die sensorischen Eindrücke nach Kontakt mit dem jeweiligen Reinigungsmittel der Probanden.

Die Mikroemulsionen werden vorzugsweise in Mengen von 0,1 bis 20 Gew.-%, vorzugsweise von 0,2 bis 10 Gew.-%, bezogen auf das Gesamtgewicht des Reinigungsmittels eingesetzt, um den gewünschten Erfolg zu erzielen.

### Beispiele

### Rezepturen für Duschgele

Die nachfolgenden Rezepturen sollen die vorliegende Erfindung verdeutlichen, ohne sie einzuschränken. Alle Mengenangaben, Anteile und Prozentanteile sind als Gewichtsprozente (Gew.-%), soweit nicht anders angegeben, auf das Gewicht und die Gesamtmenge bzw. auf das Gesamtgewicht der Zubereitungen bezogen. Die in den Beispielen angegebenen Gewichtsprozente sind Aktivgehalte bzw. Gew.-% Aktivsubstanz.

Alle Substanzen sind Produkte der Firma Cognis. Alle Substanzbezeichnungen sind eingetragene Marken.

Die erfindungsgemäße Mikroemulsion für kosmetische Reinigungsmittel - Beispielformulierung M3 bis M7

**Tabelle 1: Mikroemulsion für kosmetische Reinigungsmittel, Leitfähigkeit 0,25 mSi/cm, Viskositäten 5000 - 20000mPa s, pH = 3,2 - 4,5**

| | **Substanz** | **INCI** | **Aktivsubstanz Gew.-%** | | | | |
|---|---|---|---|---|---|---|---|
| | | | M3 | M4 | M5 | M6 | M7 |
| 1 | Cetiol 88 | Caprylylcaprylat | 38,5 | 39,0 | 42,5 | 33 | 36 |
| 2 | Plantacare 1200 UP | Lauryl Glycoside | 20 | 19,0 | 17,5 | 17,5 | 19 |
| 3 | Dehymuls PGPH | Polyglyceryl-2-dipolyhydroxystearate | 8,5 | 8,5 | 7,5 | 7,5 | 8 |
| 4 | Lameform TGI | Polyglyceryl-3-diisostearate | 8,5 | 8,5 | 7,5 | 7,5 | 8 |
| 5 | Cegesoft SB | sheabutter | 3,8 | 3,0 | 3,0 | 3,0 | 2,5 |
| | Citric Acid | | q.s. | q.s. | q.s. | q.s. | q.s. |
| 6 | Wasser | | add 100 | add 100 | add 100 | add 100 | add 100 |

Die Mikroemulsionen wurden hergestellt durch Vermischen der Komponenten unter Rühren bei einer Temperatur von 75°C. Die Viskosität wurde mit einem Brookfield Viskosimeter, Spindel 2, bei 20 rpm bestimmt.

Zur Herstellung der Duschgele wurde eine der o.g. Mikroemulsion M3 bis M7 (M4) in einem weiteren Schritt in folgende Rezeptur eingemischt. Es bildet sich eine transparente Emulsion.

**Tabelle 2: Duschgel-Rezepturen, B und C enthalten die Mikroemulsion, A, D und E dienen als Vergleichsrezeptur.**

| | **Substanz** | **INCI** | **A** | **B** | **C** | **D** | **E** |
|---|---|---|---|---|---|---|---|
| 1 | Texapon N70 | Sodium laureth sulfate + 2EO | 9 | 9 | 9 | | 10 |
| 2 | Dehyton PK45 | Cocamidopropylbetain | 3 | 3 | | 2,1 | 2 |
| 3 | Plantacare 2000UP | | | | | 7,7 | |
| | Sulfopon 1216G | | | | | 5,2 | |
| 3 | Mikroemulsion M4 (gem. Tabelle 3) | | 0 | 2,0 | 4,0 | | |
| 4 | JR-400 | PQ-10 | | | | 0,2 | |
| 5 | Jaguar C162 | Hydroxypropyl Guar, Hydroxypropyltrimoniumchlorid | 0,2 | 0,2 | 0,2 | | |
| | Guar TC | | | | | | 0,2 |
| 6 | Arlypon TT | PEG-120-PPG-10-Trimethylolpropane Trioleate | 0,6 | 0,8 | 1,0 | | 1 |
| | Rheocare XG | Xanthan | | | | 1,0 | |
| 7 | Dekafeld | DMDM Hydantoin | 0,2 | 0,2 | 0,2 | | 0,2 |
| | Sodium Benzoat | | | | | 1,0 | |
| 8 | NaCl | | 1,0 | 1,0 | 1,0 | | 1,0 |
| 9 | Wasser | | Add 100 | Add 100 | Add 100 | Add 100 | Add 100 |

### Deposition von Lipidkomponenten auf der Haut - Nachweis von Cetiol 88

Die Rezepturen F bis I wurden von Testpersonen auf ihre Eigenschaft der Deposition von Lipidkomponenten untersucht. Es sollte bestimmt werden, wie sich die Konzentration auf der Haut von definierten Lipiden nach Nutzung der erfindungsgemäßen Rezepturen im Vergleich zu Rezepturen ohne die beschriebene Mikroemulsion M3 bis M7 enthaltend Esteröle und Wachse, speziell Sheabutter verhält. Dazu wurden die Arme der Testpersonen mit 13%iger Texapon NSO-Lösung vorgewaschen, ein Blindwert bestimmt und die zu behandelnden Areale eingeteilt. Das jeweilige Areal wurde vom Testleiter mit 1 g Produkt für 45 s eingerieben und anschließend mit 950 ml Wasser definiert abgespült. Ein Glaszylinder wurde bündig aufgesetzt, 3 ml Ethanol eingefüllt und 1 min. mit einem Glasstab auf der Haut verrieben. Die Eluate wurden per Pasteur Pipette in Vials überführt und mittels GC/MS-Kopplung analysiert. Die Konzentrationen wurden in µg/cm² angegeben.

| | **Substanz** | **INCI** | **F** | **G** | **H** | **I** |
|---|---|---|---|---|---|---|
| 1 | Texapon N70 | Sodium laureth sulfate + 2EO | 9 | 9 | 9 | 9 |
| 2 | Dehyton PK45 | Cocamidopropylbetain | 3 | 3 | 3 | 3 |
| 3 | Mikroemulsion M4 (gem. Tabelle 3) | | 0 | 1,0 | 4,1 | 10,0 |
| 4 | Jaguar C162 | Hydroxypropyl Guar, Hydroxypropyltrimoniumchlorid | 0,2 | 0,2 | 0,2 | 0,2 |
| 5 | Arlypon TT | PEG-120-PPG-10-Trimethylolpropane Trioleate | 0,9 | 1,0 | 1,0 | 1,7 |
| 6 | Dekafeld | DMDM Hydantoin | 0,2 | 0,2 | 0,2 | 0,2 |
| 7 | NaCl | | 1,0 | 1,0 | 1,0 | 1,0 |
| 8 | Wasser | | Add 100 | Add 100 | Add 100 | Add 100 |
| | **Deposition von Cetiol 88** [µg/cm²] | | **0,09** | **0,41** | **1,00** | **3,73** |

Die Ergebnisse in der letzten Zeile der Tabelle zeigen deutlich, dass durch Verwendung der wachshaltigen Mikroemulsion eine deutlich höhere Deposition der Lipidkomponente zu verzeichnen ist. Diese Tests zeigen, welche positiven Effekte die wachshaltige Mikroemulsion auf die Haut hat. Die negativen Wirkungen der waschaktiven Substanzen wie beispielsweise Zerstörung des Lipidfilms auf der Haut und Austrocknung der Haut wird durch die Mitverwendung der wachshaltigen Mikroemulsion aufgehoben, ohne jedoch die positiven reinigenden Wirkungen der waschaktiven Substanzen zu verringern oder zu zerstören.

## Patentansprüche

1. Kosmetische Reinigungsmittel, enthaltend
A eine Mikroemulsion enthaltend
(a) mindestens ein Alkyl(oligo)glycosid
(b) mindestens ein von (a) unterschiedliches Co-Tensid
(c) mindestens eine nicht-wasserlösliche organische Ölkomponente
(d) mindestens ein Wachs
(e) und Wasser
B anionische Tenside
C kationische Polymere
D gegebenenfalls weitere Tenside
E gegebenenfalls kosmetische Zusatzstoffe
F Wasser.

2. Kosmetische Reinigungsmittel nach Anspruch 1, **dadurch gekennzeichnet, dass** die Mikroemulsion ein Alkyl(oligo)glycosid (a) gemäß der allgemeinen Formel R¹O-[G]ₚ enthält, in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Kosmetische Reinigungsmittel nach mindestens einem der Ansprüchen 1 bis 2, **dadurch gekennzeichnet, dass** als Co-Tensid (b) Polyolfettsäureester enthalten sind.

4. Kosmetische Reinigungsmittel nach mindestens einem der Ansprüche 1 bis 3, **dadurch gekennzeichnet, dass** als organische Ölkomponente (c) verzweigte Fettalkohole, C₆-C₂₂-Dialkylether, Esteröle, speziell Ester von verzweigten oder linearen Monocarbonsäuren mit 6 bis 22 C-Atomen mit linearen oder verzweigten Fettalkoholen mit 6 bis 22 C-Atomen, Kohlenwasserstoffe, oder deren Mischungen enthalten sind.

5. Kosmetische Reinigungsmittel nach Anspruch 4, **dadurch gekennzeichnet, dass** die in der organischen Ölkomponente (c) enthaltenden Esteröle ausgewählt sind aus der Gruppe, die gebildet wird von Estern von linearen C₆-C₂₂-Fettsäuren mit linearen oder verzweigten C₆-C₂₂-Fettalkoholen, Ester von verzweigten C₆-C₁₃-Carbonsäuren mit linearen oder verzweigten C₆-C₂₂-Fettalkoholen, Ester von linearen C₆-C₂₂-Fettsäuren mit verzweigten Alkoholen, Ester von C₁₈-C₃₈-Alkylhydroxycarbonsäuren mit linearen oder verzweigten C₆-C₂₂-Fettalkoholen, Ester von linearen und/oder verzweigten Fettsäuren mit mehrwertigen Alkoholen und/oder Guerbetalkoholen, Ester von C₆-C₂₂-Fettalkoholen und/oder Guerbetalkoholen mit aromatischen Carbonsäuren, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, lineare und verzweigte C₆-C₂₂-Fettalkoholcarbonate, Esteröle wie Isopropylpalmitat, Isopropylmyristat, Ethylhexylpalmitat, Cetylpalmitat Myristylmyristat, Oleyloleat, Ethylhexylstearate, Di-n-Octylcarbonate, Oleyloleat, Caprylylcaprylat, Oleylerucat öder deren Mischungen.

6. Kosmetische Reinigungsmittel nach mindestens einem der Ansprüche 1 bis 5, **dadurch gekennzeichnet, dass** als Wachskomponente (d) natürliche Wachse, wie z.B. Shorea Stenoptera Butter, Sheabutter, Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylengly-colwachsenatürliche und insbesondere Wachse auf Basis von Estern aus Monocarbonsäuren mit mindestens 14 C-Atomen mit Fettalkoholen mit mindestens 14 C-Atomen ausgewählt sind.

7. Kosmetische Reinigungsmittel nach mindestens einem der Ansprüche 1 bis 6, **dadurch gekennzeichnet, dass** die Mikroemulsion als zusätzliche Komponente (f) eine kationische Verbindung, insbesondere eine quaternäre Ammoniumverbindung und/oder ein kationisches Polymer enthält.

8. Kosmetische Reinigungsmittel nach mindestens einem der Ansprüche 1 bis 7, **dadurch gekennzeichnet, dass** die Mikroemulsion noch optional weitere Inhaltsstoffe als Komponente (g) enthält, die ausgewählt sind aus der Gruppe, die gebildet wird von Hydrotope wie Glycerin, Konservierungsmittel, Zitronensäure, Phenoxyethanol, UV-Lichtschutzfilter, Antioxidantien, biogene Wirkstoffe, Parfum, Farbstoffe, Biozide und pH-Regulantien.

9. Kosmetische Reinigungsmittel nach mindestens einem der Ansprüche 1 bis 8, **dadurch gekennzeichnet, dass** die Mikroemulsion
4 - 25 Gew.-% der Komponente (a)
4 - 20 Gew.-% der Komponente (b)
5 - 50 Gew.-% der Komponente (c)
0,5 - 15 Gew.-% der Komponente (d)
add 100 Gew.-% der Komponente (e)
0 - 10 Gew.-% der Komponente (f)
0 - 5 Gew.-% weitere Inhaltsstoffe (g)
enthält mit der Maßgabe, dass die Summe aus (a) bis (g) 100 ergibt.

10. Verwendung von kosmetischen Reinigungsmitteln nach Anspruch 1 als kosmetisches Reinigungsmittel in Tüchern, Duschgelen, Duschbäder, Haarshampoos, Haarlotionen, Schaumbäder, Handwaschmitteln, Gesichtsreinigern, make-up-Entfernern, Badezubereitungen, Babypflegeprodukten, Cremes, Gele, Lotionen, alkoholische und wässerig/alkoholische Lösungen, Emulsionen, Wachs/ Fett-Massen, Stiftpräparaten, Pudern oder Salben.

## Claims

1. A cosmetic cleaning agent comprising
A a microemulsion comprising
(a) at least one alkyl (oligo)glycoside
(b) at least one cosurfactant different from (a)
(c) at least one non-water-soluble organic oil component
(d) at least one wax
(e) and water
B anionic surfactants
C cationic polymers
D optionally further surfactants
E optionally cosmetic additives
F water.

2. A cosmetic cleaning agent according to claim 1, **characterized in that** the microemulsion comprises an alkyl (oligo)glycoside (a) according to the general formula R¹O-[G]p, in which R¹ is an alkyl and/or alkenyl radical having 4 to 22 carbon atoms, G is a sugar radical having 5 or 6 carbon atoms and p is numbers from 1 to 10.

3. A cosmetic cleaning agent according to at least one of claims 1 to 2, **characterized in that** polyol fatty acid esters are present as cosurfactant (b).

4. A cosmetic cleaning agent according to at least one of claims 1 to 3, **characterized in that** branched fatty alcohols, C₆-C₂₂ dialkyl ethers, ester oils, specifically esters of branched or linear monocarboxylic acids having 6 to 22 carbon atoms with linear or branched fatty alcohols having 6 to 22 carbon atoms, hydrocarbons, or mixtures thereof are present as organic oil component (c).

5. A cosmetic cleaning agent according to claim 4, **characterized in that** the ester oils present in the organic oil component (c) are selected from the group which is formed from esters of linear C₆-C₂₂ fatty acids with linear or branched C₆-C₂₂ fatty alcohols, esters of branched C₆-C₁₃ carboxylic acids with linear or branched C₆-C₂₂ fatty alcohols, esters of linear C₆-C₂₂ fatty acids with branched alcohols, esters of C₁₈-C₃₈ alkylhydroxylcarboxylic acids with linear or branched C₆-C₂₂ fatty alcohols, esters of linear and/or branched fatty acids with polyhydric alcohols and/or Guerbet alcohols, esters of C₆-C₂₂ fatty alcohols and/or Guerbet alcohols with aromatic carboxylic acids, esters of C₂-C₁₂ dicarboxylic acids with linear or branched alcohols having 1 to 22 carbon atoms or polyols having 2 to 10 carbon atoms and 2 to 6 hydroxyl groups, linear and branched C₆-C₂₂ fatty alcohol carbonates, ester oils such as isopropyl palmitate, isopropyl myristate, ethylhexyl palmitate, cetyl palmitate, myristyl myristate, oleyl oleate, ethylhexyl stearates, di-n-octyl carbonates, oleyl oleate, caprylyl caprylate, oleyl erucate or mixtures thereof.

6. A cosmetic cleaning agent according to at least one of claims 1 to 5, **characterized in that** natural waxes, such as e.g. shorea stenoptera butter, shea butter, candelilla wax, carnauba wax, Japan wax, esparto grass wax, cork wax, guaruma wax, rice germ oil wax, sugar cane wax, ouricury wax, montan wax, beeswax, shellac wax, spermaceti, lanolin (wool wax), uropygial grease, ceresine, ozokerite (earth wax), petrolatum, paraffin waxes, micro waxes; chemically modified waxes (hard waxes), such as e.g. montan ester waxes, sasol waxes, hydrogenated jojoba waxes, and synthetic waxes, such as e.g. polyalkylene waxes and polyethylene glycol waxes natural and in particular waxes based on esters of monocarboxylic acids having at least 14 carbon atoms with fatty alcohols having at least 14 carbon atoms are selected as wax component (d).

7. A cosmetic cleaning agent according to at least one of claims 1 to 6, **characterized in that** the microemulsion comprises a cationic compound, in particular a quaternary ammonium compound and/or a cationic polymer, as additional component (f).

8. A cosmetic cleaning agent according to at least one of claims 1 to 7, **characterized in that** the microemulsion also optionally comprises further ingredients as component (g) which are selected from the group which is formed from hydrotopes such as glycerol, preservatives, citric acid, phenoxyethanol, UV photoprotective filters, antioxidants, biogenic active ingredients, perfume, dyes, biocides and pH regulators.

9. A cosmetic cleaning agent according to at least one of claims 1 to 8, **characterized in that** the microemulsion comprises
4-25% by weight of component (a)
4-20% by weight of component (b)
5-50% by weight of component (c)
0.5-15% by weight of component (d)
ad 100% by weight of component (e)
0-10% by weight of component (f)
0-5% by weight of further ingredients (g),
with the proviso that the sum of (a) to (g) gives 100.

10. The use of cosmetic cleaning agents according to claim 1 as cosmetic cleaning agents in wipes, shower gels, shower baths, hair shampoos, hair lotions, foam baths, hand washing agents, face cleaners, make-up removers, bath preparations, baby care products, creams, gels, lotions, alcoholic and aqueous/alcoholic solutions, emulsions, wax/fat masses, stick preparations, powders or ointments.

## Revendications

1. Détergents cosmétiques, contenant :
A une microémulsion contenant :
(a) au moins un (oligo)glycoside d'alkyle,
(b) au moins un co-tensioactif différent de (a),
(c) au moins un composant huileux organique non soluble dans l'eau,
(d) au moins une cire,
(e) et de l'eau,
B des tensioactifs anioniques,
C des polymères cationiques,
D éventuellement d'autres tensioactifs,
E éventuellement des additifs cosmétiques,
F de l'eau.

2. Détergents cosmétiques selon la revendication 1, **caractérisés en ce que** la microémulsion contient un (oligo)glycoside d'alkyle (a) selon la formule générale R¹O-[G]ₚ, dans laquelle R¹ représente un radical alkyle et/ou alcényle de 4 à 22 atomes de carbone, G représente un radical de sucre à 5 ou 6 atomes de carbone, et p représente des nombres de 1 à 10.

3. Détergents cosmétiques selon au moins l'une quelconque des revendications 1 à 2, **caractérisés en ce que** des esters de polyols et d'acides gras sont contenus en tant que co-tensioactifs (b).

4. Détergents cosmétiques selon au moins l'une quelconque des revendications 1 à 3, **caractérisés en ce que** des alcools gras ramifiés, des éthers de dialkyle en C₆-C₂₂, des huiles d'esters, notamment des esters d'acides monocarboxyliques ramifiés ou linéaires de 6 à 22 atomes C avec des alcools gras linéaires ou ramifiés de 6 à 22 atomes C, des hydrocarbures ou leurs mélanges sont contenus en tant que composant huileux organique (c).

5. Détergents cosmétiques selon la revendication 4, **caractérisés en ce que** les huiles d'esters contenues dans le composant huileux organique (c) sont choisies dans le groupe formé par les esters diacides gras en C₆-C₂₂ linéaires avec des alcools gras en C₆-C₂₂ linéaires ou ramifiés, les esters d'acides carboxyliques en C₆-C₁₃ ramifiés avec des alcools gras en C₆-C₂₂ linéaires ou ramifiés, les esters d'acides gras en C₆-C₂₂ linéaires avec des alcools ramifiés, les esters d'acides alkylhydroxycarboxyliques en C₁₈-C₃₈ avec des alcools gras en C₆-C₂₂ linéaires ou ramifiés, les esters d'acides gras linéaires et/ou ramifiés avec des alcools polyvalents et/ou des alcools de Guerbet, les esters d'alcools gras en C₆-C₂₂ et/ou d'alcools de Guerbet avec des acides carboxyliques aromatiques, les esters d'acides dicarboxyliques en C₂-C₁₂ avec des alcools linéaires ou ramifiés de 1 à 22 atomes de carbone ou des polyols de 2 à 10 atomes de carbone et 2 à 6 groupes hydroxyle, les carbonates d'alcools gras en C₆-C₂₂ linéaires et ramifiés, les huiles d'esters telles que le palmitate d'isopropyle, le myristate d'isopropyle, le palmitate d'éthylhexyle, le palmitate de cétyle, le myristate de myristyle, l'oléate d'oléyle, le stéarate d'éthylhexyle, le carbonate de di-n-octyle, l'oléate d'oléyle, le caprylate de caprylyle, l'érucate d'oléyle ou leurs mélanges.

6. Détergents cosmétiques selon au moins l'une quelconque des revendications 1 à 5, **caractérisés en ce que** des cires naturelles, telles que p. ex. le beurre d'illipé, le beurre de karité, la cire de candelilla, la cire de carnauba, la cire du Japon, la cire de sparte, la cire de liège, la cire de guaruma, la cire d'huile de germes de riz, la cire de canne à sucre, la cire d'ouricoury, la cire de lignite, la cire d'abeille, la cire de gomme-laque, la cétine, la lanoline (cire de suint), la graisse de croupion, la cérésine, l'ozokérite (cire de terre), la vaseline, les cires de paraffine, les microcires ; des cires modifiées chimiquement (cires dures), telles que p. ex. les cires d'esters de lignite, les cires de sasol, les cires de jojoba hydrogénées, ainsi que des cires synthétiques, telles que p. ex ; les cires de polyalkylène et les cires de polyéthylèneglycol naturelles, et notamment les cires à base d'esters d'acides monocarboxyliques contenant au moins 14 atomes C avec des alcools gras contenant au moins 14 atomes C, sont choisies en tant que composant cireux (d).

7. Détergents cosmétiques selon au moins l'une quelconque des revendications 1 à 6, **caractérisés en ce que** la microémulsion contient en tant que composant supplémentaire (f) un composé cationique, notamment un composé d'ammonium quaternaire et/ou un polymère cationique.

8. Détergents cosmétiques selon au moins l'une quelconque des revendications 1 à 7, **caractérisés en ce que** la microémulsion contient encore éventuellement d'autres constituants en tant que composant (g), qui sont choisis dans le groupe formé par les hydrotopes tels que la glycérine, les conservateurs, l'acide citrique, le phénoxyéthanol, les filtres de protection contre la lumière UV, les antioxydants, les agents actifs biogènes, les parfums, les colorants, les biocides et les régulateurs de pH.

9. Détergents cosmétiques selon au moins l'une quelconque des revendications 1 à 8, **caractérisés en ce que** la microémulsion contient :
4 à 25 % en poids du composant (a),
4 à 20 % en poids du composant (b),
5 à 50 % en poids du composant (c),
0,5 à 15 % en poids du composant (d),
jusqu'à 100 % en poids du composant (e),
0 à 10 % en poids du composant (f),
0 à 5 % en poids d'autres constituants (g),
à condition que la somme de (a) à (g) soit de 100.

10. Utilisation de détergents cosmétiques selon la revendication 1 en tant que détergents cosmétiques dans des lingettes, des gels douches, des bains douches, des shampoings capillaires, des lotions capillaires, des bains moussants, des produits de nettoyage pour les mains, des produits de nettoyage pour le visage, des démaquillants, des préparations pour le bain, des produits de soin pour bébés, des crèmes, des gels, des lotions, des solutions alcooliques et aqueuses/alcooliques, des émulsions, des matières cireuses/grasses, des préparations de crayons, des poudres ou des onguents.
